# EUROPÄISCHE PATENTSCHRIFT

(11) **EP 2 667 831 B1**
(45) Veröffentlichungstag und Bekanntmachung des Hinweises auf die Patenterteilung: **09.05.2018**
(21) Anmeldenummer: 12703004.7
(22) Anmeldetag: 25.01.2012
(51) Int. Cl.: A61F 2/90

(54) **MEDIZINISCHE VORRICHTUNG MIT EINER GITTERSTRUKTUR UND BEHANDLUNGSSYSTEM MIT EINER DERARTIGEN VORRICHTUNG**
MEDICAL DEVICE HAVING A MESH STRUCTURE AND TREATMENT SYSTEM COMPRISING SUCH A DEVICE
DISPOSITIF MÉDICAL DOTÉ D'UNE STRUCTURE EN TREILLIS ET SYSTÈME DE TRAITEMENT DOTÉ D'UN TEL DISPOSITIF

(30) Priorität: 25.01.2011 DE 102011009371
(43) Veröffentlichungstag der Anmeldung: 04.12.2013
(73) Patentinhaber: Acandis GmbH & Co. KG, 76327 Pfinztal (DE)
(72) Erfinder: CATTANEO, Giorgio, 76199 Karlsruhe (DE)
(74) Vertreter: Kilchert, Jochen
(86) Internationale Anmeldenummer: PCT/EP2012/051123
(87) Internationale Veröffentlichungsnummer: WO 2012/101163

(56) Entgegenhaltungen:
- WO-A1-02/091958
- WO-A1-2006/029619
- WO-A1-2010/090348
- US-A- 5 833 699
- US-A1- 2002 147 493
- US-A1- 2011 009 950

## Beschreibung

Die Erfindung betrifft eine medizinische Vorrichtung mit einer komprimierbaren und expandierbaren, kreiszylinderförmigen Gitterstruktur, die Umfangssegmente aus geschlossenen Zellen umfasst.

Das technische Gebiet der Erfindung betrifft insbesondere stentähnliche Systeme bzw. Vorrichtungen zur Behandlung von Erkrankungen des Herz-Kreislaufsystems. Ein besonders bevorzugtes Gebiet sind Vorrichtungen zum Entfernen von Konkrementen aus Körperhohlorganen, insbesondere Thrombektomie-Devices.

Thrombektomie-Devices sind aus der Praxis beispielhaft aus US 2011/009950 A1, US 2002/147493 A1 oder WO 2006/029619 A1 bekannt und umfassen eine korbartige, insbesondere zumindest abschnittsweise kreiszylinderförmige, Gitterstruktur, die durch einen Zuführkatheter an den Behandlungsort geführt werden kann. Die Gitterstruktur ist expandierbar und komprimierbar, so dass die Gitterstruktur beim Vorschieben durch einen Zuführkatheter einen relativ kleineren Querschnittsdurchmesser als im expandierten Zustand aufweist, den die Gitterstruktur innerhalb eines Körperhohlorgans einnimmt. Die Gitterstruktur bekannter Systeme umfasst Stege, die Zellen der Gitterstruktur begrenzen. Beim Übergang vom komprimierten in den expandierten Zustand bewegen sich die Stege ausgehend von der Längsachse der Gitterstruktur radial geradlinig nach außen. Dabei können sich die Stege der bekannten Gitterstruktur in radialer Richtung bezogen auf die Längsachse der Gitterstruktur in ein Konkrement, insbesondere ein Blutgerinnsel, das in dem Körperhohlorgan angeordnet ist und beispielsweise eine Minderdurchblutung nachgeordneter Gewebeteile bewirkt, hineinschneiden. Auf diese Weise wird die bekannte Gitterstruktur mit dem Blutgerinnsel verbunden, so dass das Blutgerinnsel aus dem Körperhohlorgan extrahiert werden kann.

Es hat sich gezeigt, dass sich der Thrombus beim Entfernen des Konkrements aus dem Körperhohlorgan, insbesondere beim Ziehen der bekannten Vorrichtungen bzw. Thrombektomie-Devices durch ein Blutgefäß, von der Gitterstruktur lösen kann. Dieses Risiko besteht insbesondere beim Zurückziehen der Gitterstruktur in größere Blutgefäße, so dass sich der Thrombus in radialer Richtung nach außen von der Gitterstruktur entfernen kann. Bei den bekannten Thrombektomie-Devices besteht also die Gefahr, dass das Blutgerinnsel bzw. der Thrombus nicht ausreichend fest an der Gitterstruktur anhaftet.

Die Aufgabe der Erfindung besteht darin, eine medizinische Vorrichtung mit einer komprimierbaren und expandierbaren, kreiszylinderförmigen Gitterstruktur anzugeben, die eine verbesserte Verankerung in einem Blutgerinnsel ermöglicht und eine gute Handhabbarkeit aufweist. Ferner besteht die Aufgabe der Erfindung darin, ein Behandlungssystem mit einer derartigen Vorrichtung anzugeben.

Erfindungsgemäß wird diese Aufgabe im Hinblick auf die medizinische Vorrichtung durch den Gegenstand des Patentanspruchs 1 und im Hinblick auf das Behandlungssystem durch den Gegenstand des Patentanspruchs 12 gelöst.

Die Erfindung beruht auf dem Gedanken, eine medizinische Vorrichtung mit einer komprimierbaren und expandierbaren, kreiszylinderförmigen Gitterstruktur anzugeben, die Umfangssegmente aus geschlossenen Zellen umfasst. Die Zellen sind durch jeweils vier Stege begrenzt, die an Verbindungsstellen miteinander gekoppelt sind. Von den mindestens vier Stegen sind jeweils zwei in Umfangsrichtung der Gitterstruktur benachbarte und an einer Verbindungsstelle miteinander gekoppelte Stege unterschiedlich flexibel derart, dass der Steg mit höherer Flexibilität beim Übergang der Gitterstruktur vom expandierten Zustand in den komprimierten Zustand stärker verformbar als der Steg mit niedrigerer Flexibilität ist. Von den jeweils vier Stegen einer Zelle sind die Stege mit höherer Flexibilität und die Stege mit niedrigerer Flexibilität diagonal jeweils gegenüberliegend angeordnet derart, dass zwei in Längsrichtung der Gitterstruktur gegenüberliegend angeordnete Verbindungsstellen beim Übergang der Gitterstruktur vom expandierten Zustand in den komprimierten Zustand in Umfangsrichtung gegenläufig zueinander versetzt werden. Dabei sind alle Zellen eines Umfangssegments gleichartig ausgebildet, so dass die gesamte Gitterstruktur beim Übergang vom expandierten Zustand in den komprimierten Zustand zumindest abschnittsweise tordiert. Weiterhin sind wenigstens zwei benachbarte Umfangssegmente gleichartig ausgebildet. Die in Umfangsrichtung benachbart angeordneten Verbindungsstellen eines Umfangssegments spannen eine gemeinsame Querschnittsebene der Gitterstruktur auf, die orthogonal zur Längsachse der Gitterstruktur angeordnet ist, wobei sich die in Umfangsrichtung benachbart angeordneten Verbindungsstellen beim Übergang der Gitterstruktur vom expandierten Zustand in den komprimierten Zustand in der Querschnittsebene bewegen. Die vorgenannten Merkmale und Eigenschaften der erfindungsgemäßen medizinischen Vorrichtung werden ebenso beim Übergang der Gitterstruktur vom komprimierten zum expandierten Zustand erreicht. Mit anderen Worten gilt das Vorgenannte nicht nur für die Komprimierung der Gitterstruktur, sondern auch für die Expansion.

Gemäß dem nebengeordneten Aspekt beruht die Erfindung auf dem Gedanken, ein Behandlungssystem mit der medizinischen Vorrichtung und einem Katheter anzugeben, wobei die medizinischen Vorrichtung ein Führungselement, insbesondere einen Führungsdraht, umfasst, das mit einem axialen Ende der Gitterstruktur fest, insbesondere drehfest, verbunden und längsverschieblich in dem Katheter angeordnet ist.

Der Übergang der Gitterstruktur vom expandierten Zustand in den komprimierten Zustand, also die Komprimierung der Gitterstruktur, erfolgt vorzugsweise bei der Herstellung des Behandlungssystems, wobei die Gitterstruktur vom expandierten Herstellzustand radial komprimiert wird, um die komprimierte Gitterstruktur in den Katheter einzubringen. Durch die Komprimierung der Gitterstruktur werden die Stege der einzelnen Zellen verformt. Dabei sind die Flexibilitätseigenschaften bzw. allgemein die Flexibilität der einzelnen Stege unterschiedlich eingestellt, so dass sich die einzelnen Stege innerhalb der Gitterstruktur bei der Komprimierung unterschiedlich flexibel verhalten. Insbesondere sind die Flexibilitätseigenschaften der einzelnen Stege einer Zelle derart eingestellt, dass sich die gesamte Gitterstruktur bei der Komprimierung verdreht bzw. tordiert. Dieser Effekt tritt auch bei der Expansion der Gitterstruktur, also beim Übergang der Gitterstruktur vom komprimierten in den expandierten Zustand, auf. Dabei wird die Expansion vorzugsweise beim Entlassen der komprimierten Gitterstruktur aus dem Katheter bewirkt. Die Expansion kann selbsttätig durch selbstexpandierbare Eigenschaften der Gitterstruktur erfolgen. Durch Wegfall des äußeren Komprimierungszwangs, der durch den Katheter auf die Gitterstruktur ausgeübt wird, werden die Radialkräfte der Gitterstruktur frei, so dass sich die Gitterstruktur in radialer Richtung aufweitet. Dabei bewirken die unterschiedlichen Flexibilitätseigenschaften der einzelnen Stege ein unterschiedliches Verhalten der einzelnen Stege bei der Expansion. Insbesondere sind die Flexibilitätseigenschaften derart eingestellt, dass sich die gesamte Gitterstruktur bei der Expansion verdreht bzw. um die Längsachse tordiert. Dadurch wird erreicht, dass sich die einzelnen Stege der Gitterstruktur bei der Expansion nicht nur in radiale Richtung bewegen, sondern zumindest segmentweise gleichzeitig eine Bewegungskomponente in Umfangsrichtung aufweisen. Dies gilt insbesondere für die Verbindungsstellen, die sich bei der Expansion der Gitterstruktur nicht nur in radialer Richtung von der Längsachse entfernen, sondern auch segmentweise eine Drehbewegung in Umfangsrichtung aufweisen. Die Drehbewegung kann segmentweise unterschiedlich stark ausgeprägt sein. Insbesondere kann die Drehbewegung derart ausgebildet sein, dass sich die in Längsrichtung beabstandeten Verbindungsstellen derart in Umfangsrichtung verdrehen, dass sich entlang der gesamten Gitterstruktur im Wesentlichen eine schraubenförmige Verdrehung bzw. Torsion einstellt.

Der Vorteil, der mit dem zuvor beschriebenen Expansionsmechanismus erreicht wird, besteht darin, dass sich die Gitterstruktur beim Aufweiten in einem Blutgefäß nicht nur radial, sondern auch in Umfangsrichtung in ein Blutgerinnsel einschneidet. Dadurch werden innerhalb des Blutgerinnsels Hinterschnitte ausgebildet, so dass die Verankerung der Gitterstruktur im Blutgerinnsel bzw. allgemein die Verbindung zwischen Gitterstruktur und Blutgerinnsel verbessert ist. Die Gefahr der Ablösung des Blutgerinnsels beim Entfernen aus einem Blutgefäß wird somit reduziert. Da die Stege der Gitterstruktur sich nicht nur in radialer Richtung, sondern auch gleichzeitig in Umfangsrichtung in das Blutgerinnsel einschneiden, wird der Weg der einzelnen Stege durch das Material des Blutgerinnsels verlängert. Dies trägt zur verbesserten Verankerung der Gitterstruktur im Blutgerinnsel bei. Insgesamt weist die erfindungsgemäße Vorrichtung also verbesserte Schneideigenschaften der Gitterstruktur auf, die zu einer verbesserten Arretierung der Gitterstruktur in einem Thrombus bzw. allgemein Blutgerinnsel führen.

Da die in Umfangsrichtung benachbart angeordneten Verbindungsstellen eines Umfangssegments eine gemeinsame Querschnittsebene der Gitterstruktur aufspannen, die orthogonal zur Längsachse der Gitterstruktur angeordnet ist, bewegen sich die in Umfangsrichtung benachbart angeordneten Verbindungsstellen beim Übergang der Gitterstruktur vom expandierten Zustand in den komprimierten Zustand in der Querschnittsebene. Mit anderen Worten ist vorgesehen, dass in Umfangsrichtung beabstandet angeordnete Verbindungsstellen in jedem Zustand der Gitterstruktur zueinander eine Relativposition einnehmen, die durch eine orthogonale Ebene zur Längsachse der Gitterstruktur beschrieben ist. Während die in Längsrichtung beabstandet angeordneten Verbindungsstellen beim Übergang der Gitterstruktur vom komprimierten in den expandierten Zustand und umgekehrt, zueinander in Umfangsrichtung versetzt werden, erfolgt in Längsrichtung zwischen zwei in Umfangsrichtung benachbart angeordneten Verbindungsstellen kein Versatz. Vielmehr weisen die einzelnen Umfangssegmente ringförmig auf dem Umfang der Gitterstruktur angeordnete Verbindungsstellen auf, die zueinander fluchten. Die in Umfangsrichtung benachbarten Verbindungsstellen fluchten dabei in jedem Zustand der Gitterstruktur, insbesondere im komprimierten und im expandierten Zustand.

Die von den in Umfangsrichtung benachbart angeordneten Verbindungsstellen aufgespannte Querschnittsebene liegt in jedem Zustand der Expansion bzw. Komprimierung der Gitterstruktur vor, auch wenn sich die Querschnittsebene bei der Expansion oder der Komprimierung der Gitterstruktur insgesamt in Längsrichtung verschiebt. Eine derartige Verschiebung kann beispielweise durch das so genannte Foreshortening bewirkt sein, wobei sich die Gitterstruktur insgesamt verkürzt, wenn eine radiale Expansion erfolgt.

Durch die Anordnung der Verbindungsstellen auf einer gemeinsamen Querschnittsebene ist sichergestellt, dass sich die in Längsrichtung benachbart angeordneten Verbindungsstellen beim Übergang der Gitterstruktur vom komprimierten in den expandierten Zustand und umgekehrt zueinander versetzen. Insbesondere können sich die Verbindungsstellen, die in Längsrichtung der Gitterstruktur gegenüberliegend angeordnet und einer gemeinsamen Zelle zugeordnet sind, beim Übergang der Gitterstruktur vom expandierten Zustand in den komprimierten Zustand und umgekehrt gegenläufig in Umfangsrichtung der Gitterstruktur bewegen. Beispielsweise kann sich eine weiter proximal angeordnete Verbindungsstelle bei der Zustandsänderung der Gitterstruktur in Uhrzeigerrichtung bzw. in positiver Umlaufrichtung um die Längsachse der Gitterstruktur drehen, wogegen eine weiter distal angeordnete Verbindungsstelle bei derselben Zustandsänderung der Gitterstruktur eine Bewegung in Gegenuhrzeigerrichtung, also in negativer Umlaufrichtung, durchführen kann.

Bei einer weiteren bevorzugten Ausführungsform der erfindungsgemäßen Vorrichtung umfasst jede Zelle vier Verbindungsstellen, die im expandierten Zustand der Gitterstruktur eine drachenviereckförmige Grundform der Zelle aufspannen. Konkret bilden die gedachten Geraden zwischen den durch Stege miteinander verbundenen Verbindungsstellen einer Zelle vorzugsweise ein Drachenviereck, besonders vorzugsweise eine Raute. Eine derartige Grundform der geschlossenen Zelle ist für die Verdrehfunktion bzw. Torsionsfunktion der Gitterstruktur besonders vorteilhaft.

Die unterschiedlichen Flexibilitäten der einzelnen Stege der Zelle können auf verschiedene Art und Weise eingestellt werden. Insbesondere können die unterschiedlichen Flexibilitäten der einzelnen Stege, insbesondere die Flexibilitätsunterschiede zwischen den Stegen mit höherer Flexibilität und den Stegen mit niedrigerer Flexibilität, durch eine der im Folgenden erläuterten konstruktiven Ausführungsformen erreicht werden, wobei beliebige Kombinationen der genannten Ausführungsformen möglich sind.

Bei einer bevorzugten Ausführungsform wird die unterschiedliche Flexibilität der einzelnen Stege einer Zelle dadurch erreicht, dass die in Längsrichtung benachbart angeordneten Stege einer Zelle jeweils unterschiedliche Steglängen aufweisen. Dabei weisen die Stege, die gegenüber den in Längsrichtung benachbart angeordneten Stegen eine größere Steglänge aufweisen, eine größere Flexibilität auf. Das bedeutet, dass sich die Stege mit größerer Steglänge, also die Stege mit höherer Flexibilität, bei der Zustandsänderung der Gitterstruktur stärker verformen als die Stege mit kleinerer Steglänge, also die Stege mit kleinerer Flexibilität.

Alternativ oder zusätzlich können die Stege mit höherer Flexibilität im Wesentlichen S-förmig ausgebildet sein. Die Stege mit niedrigerer Flexibilität sind hingegen geradlinig ausgebildet. Die S-förmige Ausbildung der Stege bewirkt, dass derartige Stege insgesamt eine höhere Steglänge aufweisen als gleichwertige geradlinige Stege. Somit wird die höhere Flexibilität einerseits durch die tatsächlich längere Steglänge und andererseits durch die geometrische Form der Stege bewirkt. Die geometrische Form trägt insofern zur Flexibilität bei, als durch die S-förmige Krümmung eine stärkere Verformung, also eine stärkere Bewegung zwischen den Endpunkten des S-förmig gebogenen Steges beim Übergang der Gitterstruktur vom expandierten in den komprimierten Zustand und umgekehrt erreicht wird.

Die Stege mit höherer Flexibilität können auch eine Stegbreite aufweisen, die kleiner als die Stegbreite der Stege mit niedrigerer Flexibilität ist. Die Stege mit kleinerer Stegbreite wirken einer Verformung, die durch die Expansion oder Komprimierung der Gitterstruktur bewirkt wird, weniger stark entgegen, so dass die Stege mit kleinerer Stegbreite insgesamt eine höhere Flexibilität aufweisen. Die Stege mit kleinerer Stegbreite lassen sich also stärker verformen, als die relativ steiferen Stege mit größerer Stegbreite.

Eine weitere zusätzliche oder alternative Möglichkeit zur Einstellung der Flexibilität der einzelnen Stege besteht darin, dass die Stege mit höherer Flexibilität mit Biegestellen, insbesondere Verjüngungen oder Verbreiterungen oder Durchbrüchen, versehen sind. Beispielsweise können die Stege mit höherer Flexibilität Einkerbungen, also lokal begrenzte Stellen, die sich gegenüber dem Gesamtsteg durch eine Materialreduzierung auszeichnen, aufweisen. Die Biegestellen können dabei im Wesentlichen Sollbiegestellen oder Knickpunkte bilden, an denen der Steg vergleichsweise leicht gebogen werden kann. Die Biegestellen bewirken also eine höhere Flexibilität der Stege gegenüber den Stegen mit relativ niedrigerer Flexibilität. Die Verjüngungen bilden dabei Teilbereiche des Stegs, die beispielsweise eine kleinere Stegbreite also die restlichen Bereiche desselben Stegs und insbesondere als die weiteren Stege mit niedrigerer Flexibilität aufweisen. Die Biegestellen können auch als Durchbrüche, beispielsweise als Fenster innerhalb eines Stegs ausgebildet sein. Der Steg kann also lokal Öffnungen aufweisen, so dass die Materialstärke des Stegs stellenweise reduziert ist. Dadurch wird erreicht, dass die Flexibilität des Stegs erhöht ist. Gegenüber Stegen, die derartige Durchbrüche nicht aufweisen, weist der Steg mit einem Durchbruch somit eine höhere Flexibilität auf.

Zweckmäßigerweise ist bei der erfindungsgemäßen medizinischen Vorrichtung vorgesehen, dass die Stege an den Verbindungsstellen einstückig miteinander verbunden sind. Eine Relativbewegung der Stege untereinander wird somit unterbunden. Vielmehr erfolgt die Verformung der gesamten Gitterstruktur beim Übergang vom komprimierten Zustand in den expandierten Zustand und umgekehrt durch eine Verbiegung bzw. flexible Auslenkung der einzelnen Stege. Ein Gleiten von Gitterelementen übereinander, wie beispielsweise bei Gittergeflechten vorgesehen, ist nicht möglich.

An den Verbindungsstellen sind vorzugsweise jeweils vier Stege miteinander verbunden.

Gemäß einer weiteren bevorzugten Ausführungsform der erfindungsgemäßen medizinischen Vorrichtung ist vorgesehen, dass die in Längsrichtung der Gitterstruktur gegenüberliegend angeordneten Verbindungsstellen einen unterschiedlichen Versetzungsgrad beim Übergang der Gitterstruktur vom expandierten in den komprimierten Zustand aufweisen. Zur Bestimmung des Versetzungsgrades wird zunächst ein Nullpunktzustand festgelegt. Vorzugsweise entspricht der Nullpunktzustand dem Zustand der Gitterstruktur, in dem die in Längsrichtung gegenüberliegend angeordneten Verbindungsstellen zueinander nicht versetzt sind, also in einer Linie fluchten, die sich parallel zur Längsachse der Gitterstruktur erstreckt. Bei einer Zustandsänderung der Gitterstruktur, beispielsweise bei einer Komprimierung oder Expansion ausgehend vom Nullpunktzustand, werden die in Längsrichtung benachbarten Verbindungsstellen aus der Nullpunktlage ausgelenkt. Die in Längsrichtung benachbart angeordneten bzw. gegenüberliegend angeordneten Verbindungsstellen versetzen sich zueinander. Der Grad der Auslenkung bzw. des Versatzes kann unterschiedlich sein. Beispielsweise kann eine weiter proximal angeordnete Verbindungsstelle einer Zelle bei der Zustandsänderung der Gitterstruktur weiter aus der Nullpunktlage ausgelenkt bzw. versetzt werden, als eine weiter distal angeordnete Verbindungsstelle einer Zelle. Der Versetzungsgrad von in Umfangsrichtung benachbart angeordneten Verbindungsstellen eines Umfangssegments bleibt jedoch in jedem Zustand der Gitterstruktur gleich. Mit anderen Worten entspricht der Umfang eines Umfangssegments in jedem Zustand der Gitterstruktur einem Vielfachen des Abstands zwischen zwei in Umfangsrichtung benachbart angeordneten Verbindungsstellen des Umfangssegments.

Ein Teil der Gitterstruktur kann beim Übergang vom komprimierten Zustand in den expandierten Zustand und umgekehrt in positiver Drehrichtung tordieren und ein weiterer, in Längsrichtung der Gitterstruktur nachgeordneter Teil in negativer Drehrichtung. Insgesamt kann die Gitterstruktur mehrere Abschnitte aufweisen, die in unterschiedliche Richtungen tordieren. Dadurch kann die Verankerung eines Blutgerinnsels bzw. Thrombus mit der Gitterstruktur weiter verbessert werden.

Die Umfangssegmente können jeweils zwei Teilsegmente umfassen, die jeweils mäanderförmig angeordnete Stege aufweisen. Dabei können die in Längsrichtung der Gitterstruktur unmittelbar benachbarten Teilsegmente zumindest im komprimierten Zustand der Gitterstruktur unterschiedliche Teilsegmentbreiten aufweisen. Mit anderen Worten können die in Umfangsrichtung benachbart bzw. gegenüberliegend angeordneten Verbindungsstellen eines Umfangssegments das Umfangssegment in zwei Teilsegmente trennen, wobei ein Teilsegment breiter als das andere Teilsegment ist. Dies entspricht im Wesentlichen einer drachenförmigen Grundform der Zellen des Umfangssegments. Bei einer rautenförmigen Grundform der Zellen des Umfangssegments, die einen Spezialfall der drachenförmigen Grundform darstellt, teilt die Verbindungslinie zwischen zwei in Umfangsrichtung gegenüberliegend angeordneten Verbindungsstellen das Umfangssegment in zwei gleichbreite Teilsegmente.

Die Erfindung wird im Folgenden anhand von Ausführungsbeispielen unter Bezugnahme auf die beigefügten, schematischen Zeichnungen näher erläutert. Darin zeigen
- Fig. 1: eine Draufsicht auf eine Zelle der Gitterstruktur der erfindungsgemäßen medizinischen Vorrichtung nach einem bevorzugten Ausführungsbeispiel im expandierten Zustand;
- Fig. 2: die Zelle gemäß Fig. 1 im komprimierten Zustand;
- Fig. 3: einen Ausschnitt eines Umfangssegments mit mehreren Zellen gemäß Fig. 1 im komprimierten Zustand;
- Fig. 4: einen Querschnitt durch ein Blutgefäß mit einem Thrombus und der darin angeordneten Gitterstruktur der erfindungsgemäßen medizinischen Vorrichtung im Gebrauch;
- Fig. 5: eine schematische Darstellung zur Erläuterung der Messung unterschiedlicher Flexibilitäten bei den Stegen der Gitterstruktur der erfindungsgemäßen medizinischen Vorrichtung;
- Fig. 6: die schematische Darstellung gemäß Fig. 5, wobei verdeutlicht ist, wie das Verhalten der Stege von der Relativposition der Verbindungsstellen abhängig ist;
- Fig. 7: eine Draufsicht auf eine Zelle der Gitterstruktur der erfindungsgemäßen medizinischen Vorrichtung gemäß einem bevorzugten Ausführungsbeispiel im expandierten Zustand der Gitterstruktur, wobei alle Stege der Zelle unterschiedliche Formen bzw. Steglängen aufweisen;
- Fig. 8: die Zelle gemäß Fig. 7 im komprimierten Zustand der Gitterstruktur;
- Fig. 9: einen Ausschnitt aus zwei benachbarten Umfangssegmenten mit jeweils mehreren Zellen gemäß Fig. 7 im expandierten Zustand; und
- Fig. 10: die Umfangssegmente gemäß Fig. 9 im komprimierten Zustand.

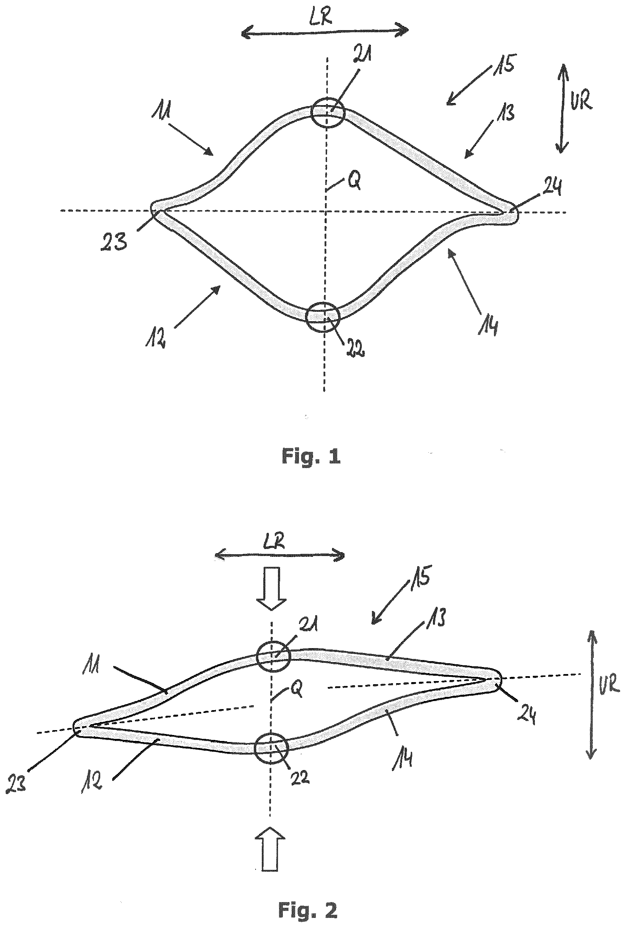

Fig. 1 zeigt eine freigeschnittene Zelle 15 einer Gitterstruktur 10 der erfindungsgemäßen medizinischen Vorrichtung gemäß einem bevorzugten Ausführungsbeispiel. Im Allgemeinen weist die medizinische Vorrichtung eine Gitterstruktur auf, die eine Vielzahl von Zellen 15 umfasst. Die Gitterstruktur 10 ist einstückig ausgebildet, insbesondere einstückig aus einem Vollmaterial durch Ausschneiden der Zellöffnungen hergestellt. Vorzugsweise ist die Gitterstruktur 10 durch Laserschneiden hergestellt bzw. bildet eine lasergeschnittene Gitterstruktur 10. Die Gitterstruktur 10 ist zumindest abschnittsweise kreiszylinderförmig ausgebildet. Das bedeutet, dass die Gitterstruktur 10 eine Wandungsebene bildet, die eine kreiszylindrische Form aufweist. Mit anderen Worten kann die Gitterstruktur 10 zumindest abschnittsweise röhrchenartig, insbesondere stentartig, ausgebildet sein.

In diesem Zusammenhang wird darauf hingewiesen, dass sich die nachfolgende Detailbeschreibung der Gitterstruktur 10 auf den Herstellzustand, also den vollständig expandierten Zustand der Gitterstruktur 10 bezieht, soweit nichts anderes angegeben ist. Der Bezugspunkt für die medizinischen Richtungsangaben "proximal" und "distal" ist der Anwender der medizinischen Vorrichtung bzw. des Behandlungssystems. Proximal angeordnete Elemente sind dem Anwender der Vorrichtung bzw. des Behandlungssystems also näher als distal angeordnete Elemente.

Die Gitterstruktur 10 weist mehrere Umfangssegmente 20 auf, die durch mehrere geschlossene Zellen 15 gebildet sind. Eine Reihe von geschlossenen Zellen 15 erstreckt sich in Umfangsrichtung UR um die Längsachse der Gitterstruktur 10 und bildet einen Zellenring, der im Rahmen der Anmeldung als Umfangssegment 20 bezeichnet wird. Mehrere in axialer Richtung bzw. in Längsrichtung der Gitterstruktur 10 benachbarte und miteinander verbundene Umfangssegmente 20 bilden die Gitterstruktur 10.

Die einzelnen Zellen, wie beispielsweise die Zelle gemäß Fig. 1, sind durch jeweils vier Stege 11, 12, 13, 14 begrenzt. Die Stege 11, 12, 13, 14 sind an vier Verbindungsstellen 21, 22, 23, 24 miteinander fest verbunden, insbesondere einstückig verbunden. Die Zelle 15 weist im Wesentlichen eine drachenviereckförmige Grundform auf. Das bedeutet, dass die Verbindungsstellen 21, 22, 23, 24 die Eckpunkte eines Drachenvierecks bilden, wobei sich die Stege 11, 12, 13, 14 im Wesentlichen entlang der Seiten des Drachenvierecks erstrecken und die Verbindungsstellen 21, 22, 23, 24 miteinander verbinden. Dabei kann die Form der Stege 11, 12, 13, 14 von der geraden Verbindungsstrecke zwischen zwei Verbindungsstellen 21, 22, 23, 24 abweichen, also beispielsweise eine gekrümmte oder gebogene Form bilden. Die Grundform eines Drachenvierecks bleibt jedoch erkennbar. Die Zelle 15 umfasst einen ersten Steg 11, einen zweiten Steg 12, einen dritten Steg 13 und einen vierten Steg 14. Der erste Steg 11 erstreckt sich zwischen einer ersten Verbindungsstelle 21 und einer dritten Verbindungsstelle 23. Der zweite Steg 12 verbindet die dritte Verbindungsstelle 23 mit einer zweiten Verbindungsstelle 22. Zwischen der zweiten Verbindungsstelle 22 und einer vierten Verbindungsstelle 24 erstreckt sich der vierte Steg 14. Der dritte Steg 13 ist zwischen der ersten Verbindungsstelle 21 und der vierten Verbindungsstelle 24 angeordnet. Der erste Steg 11 ist bezüglich des dritten Stegs 13 in Längsrichtung LR der Gitterstruktur 10 benachbart angeordnet. Bezüglich des zweiten Stegs 12 ist der erste Steg 11 in Umfangsrichtung UR der Gitterstruktur 10 benachbart angeordnet. Der dritte Steg 13 und der vierte Steg 14 sind ebenfalls in Umfangsrichtung UR der Gitterstruktur 10 benachbart angeordnet. In Längsrichtung LR der Gitterstruktur 10 benachbart angeordnet sind auch der zweite Steg 12 und der vierte Steg 14.

Die Zelle 15 gemäß Fig. 1 liegt im expandierten Zustand der Gitterstruktur 10 vor. Dabei ist zu erkennen, dass die erste Verbindungsstelle 21 und die zweite Verbindungsstelle 22 eine Verbindungslinie aufweisen, die sich quer durch die Zelle 15 erstreckt und in Umfangsrichtung UR der Gitterstruktur 10 verläuft. Konkret bildet die Verbindungslinie zwischen den in Umfangsrichtung gegenüberliegend angeordneten Verbindungsstellen 21, 22 eine Kreislinie, die sich um den Umfang der Gitterstruktur 10 erstreckt. Insgesamt sind die in Umfangsrichtung UR gegenüberliegend angeordneten Verbindungsstellen 21, 22 eines Umfangssegments 20 der Gitterstruktur 10 in einer gemeinsamen Ebene Q angeordnet, die orthogonal auf der Längsachse der Gitterstruktur 10 steht. Die gemeinsame Querschnittsebene Q, die alle in Umfangsrichtung UR benachbart bzw. gegenüberliegend angeordneten Verbindungsstellen 21, 22 eines Umfangssegments 20 umfasst, ist in den Fig. 1, 2, 7, 8 und 9 durch eine vertikal in der Zeichnungsebene angeordnete gestrichelte Linie dargestellt.

In Fig. 1 ist ferner zu erkennen, dass die in Längsrichtung LR der Gitterstruktur 10 gegenüberliegend angeordneten Verbindungsstellen 23, 24, die durch die dritte und vierte Verbindungsstelle 23, 24 gebildet sind, auf einer Linie angeordnet sind, die sich in der Zeichnungsebene horizontal erstreckt und als gestrichelte Linie dargestellt ist.

Die Verbindungslinie zwischen den dritten und vierten Verbindungsstellen 23, 24 erstreckt sich im expandierten Zustand der Gitterstruktur 10 gemäß Fig. 1 parallel zur Längsachse der Gitterstruktur 10. Die Verbindungslinie zwischen den dritten und vierten Verbindungsstellen 23, 24 steht also senkrecht auf der Querschnittsebene Q.

Die Stege 11, 12, 13, 14 der Zelle 15 weisen unterschiedliche Flexibilitäten auf. Insbesondere ist vorgesehen, dass jeweils zwei in Umfangsrichtung UR benachbart angeordnete Stege 11, 12 bzw. 13, 14, die jeweils eine gemeinsame Verbindungsstelle 23, 24 aufweisen, unterschiedlich flexibel sind. Dabei ist die Flexibilität der einzelnen Stege 11, 12, 13, 14 derart eingestellt, dass jeweils zwei bezogen auf die Zelle 15 gegenüberliegend angeordnete Stege 11, 14 bzw. 12, 13 eine höhere Flexibilität als die beiden anderen gegenüberliegenden Stege 12, 13 bzw. 11, 14 aufweisen. Bei dem Ausführungsbeispiel gemäß Fig. 1 ist insbesondere vorgesehen, dass der erste Steg 11 und der vierte Steg 14 jeweils eine höhere Flexibilität aufweisen als der zweite Steg 12 und der dritte Steg 13. Dabei kann sich die Flexibilität des ersten Stegs 11 von der Flexibilität des vierten Stegs 14 unterscheiden. Dasselbe gilt für den zweiten Steg 12 und den dritten Steg 13. Die Flexibilität des zweiten Stegs 12 und des dritten Stegs 13 kann also unterschiedlich sein. Zweckmäßig ist es, wenn der zweite Steg 12 und der dritte Steg 13 jeweils eine niedrigere Flexibilität als der erste Steg 11 und der vierte Steg 14 aufweisen.

Als Flexibilität wird im Rahmen der Anmeldung das Vermögen eines Stegs 11, 12, 13 bezeichnet, sich beim Übergang vom expandierten Zustand in den komprimierten Zustand zu verformen bzw. zu biegen. Der Grad der Auslenkung des Stegs 11, 12, 13, 14 aus dem Ruhezustand bei Einwirkung einer vorbestimmten Kraft bestimmt die Höhe der Flexibilität. Eine größere Auslenkung eines Stegs 11, 12, 13, 14 bedeutet eine höhere Flexibilität. Mit anderen Worten ist ein Steg 11, 12, 13, 14 flexibler als ein anderer Steg 11, 12, 13, 14, wenn sich der betreffende Steg 11, 12, 13, 14 unter Einwirkung derselben Kraft stärker verformt bzw. stärker ausgelenkt wird als der andere Steg 11, 12, 13, 14.

Die Flexibilität eines Stegs 11, 12, 13, 14 gegenüber anderen Stegen 11, 12, 13, 15 kann auf unterschiedliche Weise eingestellt werden. Einerseits kann die Flexibilität des Stegs 11, 12, 13, 14 dadurch erhöht werden, dass der Steg 11, 12, 13, 14 nicht geradlinig, sondern gekrümmt ausgebildet ist. Diese Variante der Erhöhung der Flexibilität ist bei dem Ausführungsbeispiel gemäß Fig. 1 und 2 beim ersten Steg 11 und beim vierten Steg 14 verwirklicht. Der erste Steg 11 und der vierte Steg 14 weisen konkret eine S-förmige Krümmung auf bzw. erstrecken sich S-förmig zwischen den Verbindungsstellen 21, 22, 23, 24 und weisen eine relativ höhere Flexibilität auf. Der zweite Steg 12 und der dritte Steg 13 sind hingegen geradlinig ausgebildet und weisen daher eine relativ niedrigere Flexibilität auf.

Eine weitere Möglichkeit, die Flexibilität eines Stegs 11, 12, 13, 14 einzustellen, besteht darin, die Steglänge zu variieren. Ein längerer Steg 11, 12, 13, 14 weist eine höhere Flexibilität als ein kürzerer Steg 11, 12, 13, 14 auf. Dies ist bei dem Ausführungsbeispiel gemäß Fig. 1 und 2 ebenfalls verwirklicht. Insbesondere ist der vierte Steg 14, der im Wesentlichen die gleiche Form wie der erste Steg 11 aufweist, länger als der erste Steg 11. Der dritte Steg 13 ist außerdem länger als der zweite Steg 12. Der vierte Steg 14 weist daher eine höhere Flexibilität als der erste Steg 11 auf. Der dritte Steg 13 weist eine höhere Flexibilität als der zweite Steg 12 auf. Insgesamt sind die Flexibilitäten des ersten Stegs 11 und des vierten Stegs 14 jedoch höher als die Flexibilitäten des zweiten Stegs 12 und des dritten Stegs 13. Konkret weist der erste Steg 11 eine höhere Flexibilität als der dritte Steg 13 auf.

Grundsätzlich ist es auch möglich, dass der erste Steg 11 und der dritte Steg 13, also allgemein in Längsrichtung LR der Gitterstruktur 10 benachbart angeordnete Stege 11, 13 und 12, 14 jeweils die gleiche Flexibilität aufweisen. Wesentlich ist, das in Umfangsrichtung UR der Gitterstruktur 10 benachbart angeordnete und miteinander verbundene Stege 11, 12 und 13, 14 unterschiedlich flexibel sind.

Eine weitere Variante zur Änderung der Flexibilität eines Stegs 11, 12, 13, 14 ist eine Variation der Stegbreite. Im Allgemeinen weist ein Steg 11, 12, 13, 14 mit relativ kleinerer Stegbreite eine höhere Flexibilität als ein Steg 11, 12, 13, 14 mit relativ größerer Stegbreite auf. Durch Variation der Stegbreite kann also die Flexibilität unterschiedlicher Stege 11, 12, 13, 14 eingestellt werden. Ferner kann die Flexibilität der Stege 11, 12, 13, 14 durch den Einsatz von Biegestellen eingestellt werden. Derartige Biegestellen können an beliebiger Stelle eines Stegs 11, 12, 13, 14 eingebracht sein und beispielsweise Verjüngungen, also lokale Änderungen der Stegbreite, umfassen. Es ist auch denkbar, die Biegestellen durch Verbreiterungen des Stegs 11, 12, 13, 14 zu realisieren. Ferner können die Biegestellen als Fenster bzw. Durchbrüche gebildet sein. Beispielsweise kann ein Steg 11, 12, 13, 14 gezielt mit Bohrungen bzw. Löchern versehen sein, so dass eine Biegestelle bzw. eine Knickstelle bereitgestellt wird. Durch eine gezielte stellenweise Materialreduktion können also Biegestellen bereitgestellt werden, die der Verformung des Stegs 11, 12, 13, 14 beim Übergang der Gitterstruktur vom expandierten Zustand in den komprimierten Zustand frühzeitig nachgeben, so dass sich der Steg 11, 12, 13, 14 insgesamt stärker verformt. Der Steg 11, 12, 13, 14 weist somit eine höhere Flexibilität auf.

Wie zuvor erläutert, weist die Zelle 15 im Wesentlichen eine drachenviereckförmige Grundform auf. Die beiden Verbindungsgeraden zwischen der ersten Verbindungsstelle 21 und der zweiten Verbindungsstelle 22 sowie der dritten Verbindungsstelle 23 und der vierten Verbindungsstelle 24 halbieren sich also nicht gegenseitig, wie es beispielsweise bei einer Raute der Fall ist. Im Ruhezustand gemäß Fig. 1 halbiert die in Längsrichtung LR ausgerichtete Verbindungsgerade zwischen der dritten und vierten Verbindungsstelle 23, 24 die in Umfangsrichtung ausgerichtete Verbindungsgerade zwischen der ersten und zweiten Verbindungsstelle 21, 22. Umgekehrt ist dieses Teilungsverhältnis nicht zwingend erfüllt. Im speziellen Fall, in dem die Grundform der Zelle 15 eine Raute bildet halbiert auch die Verbindungsgerade zwischen der ersten und zweiten Verbindungsstelle 21, 22 die Verbindungsgerade zwischen der dritten und vierten Verbindungsstelle 23, 24. Die beiden Verbindungsgerade halbieren sich also im Falle einer rautenförmigen Grundform der Zelle 15 gegenseitig, wogegen bei einer drachenviereckförmigen Grundform nur eine der beiden Verbindungsgeraden durch die andere halbiert wird.

Bei der Komprimierung der Gitterstruktur 10 verschieben sich die erste Verbindungsstelle 21 und die zweite Verbindungsstelle 22 in der Querschnittsebene Q und nähern sich dabei an. Der auf die Längsachse der Gitterstruktur 10 bezogene Radius der Kreislinie, die die Verbindungsstellen 21, 22 eines Umfangssegments verbindet, reduziert sich. Aufgrund der unterschiedlichen Flexibilitäten der Stege 11, 12, 13, 14 werden die in Längsrichtung LR der Gitterstruktur 10 gegenüberliegend angeordneten Verbindungsstellen 23, 24 zueinander versetzt bzw. ausgelenkt. Insbesondere bewirkt die höhere Flexibilität des ersten Stegs 11 und des vierten Stegs 14, dass die dritte Verbindungsstelle 23 gegenläufig zur vierten Verbindungsstelle 24 um die Längsachse der Gitterstruktur 10 rotiert. In Fig. 2 ist gut zu erkennen, dass die dritte Verbindungsstelle 23 und die vierte Verbindungsstelle 24 gegeneinander verdreht bzw. versetzt sind.

In Fig. 2 sind ferner die Winkelhalbierenden durch die Verbindungsstellen 23, 24 durch gestrichelte Linien eingezeichnet. Es ist zu erkennen, dass die Winkelhalbierenden zwischen den in Umfangsrichtung UR benachbart angeordneten und miteinander verbundenen Stege 11, 12 und 13, 14 keine gemeinsame Gerade bilden, sondern bezüglich einer Horizontalen unterschiedliche Winkel aufweisen. Daraus ist zu erkennen, dass die Flexibilitäten der einzelnen Stege 11, 12, 13, 14 derart unterschiedlich sind, dass der Versetzungsgrad der in Längsrichtung gegenüberliegend angeordneten Verbindungsstellen 23, 24 unterschiedlich ist. Konkret wird bei dem Ausführungsbeispiel gemäß Fig. 1 und 2 die vierte Verbindungsstelle 24 beim Übergang der Gitterstruktur vom expandierten Zustand in den komprimierten Zustand weiter aus der ursprünglichen Lage gemäß Fig. 1 ausgelenkt als die dritte Verbindungsstelle 23.

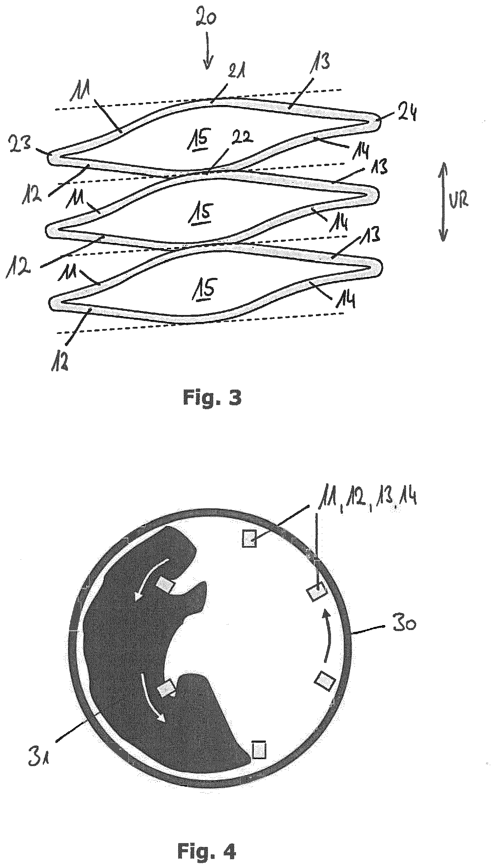

Um die Funktion der medizinischen Vorrichtung, insbesondere die Rotation der gesamten Gitterstruktur 10, zu gewährleisten, weist jedes Umfangssegment 20 gleichartig ausgebildete Zellen 15 auf. Die Zellenform wiederholt sich also innerhalb eines Umfangssegments 20. Die einzelnen Zellen 15 eines Umfangssegments 20 sind dabei in den in Umfangsrichtung UR gegenüberliegend angeordneten Verbindungsstellen 21, 22 miteinander fest verbunden, insbesondere einstückig verbunden. Ein Ausschnitt eines Umfangssegments 20 mit drei miteinander gekoppelten Zellen 15 ist in Fig. 3 dargestellt.

Die Funktion der Gitterstruktur 10 der medizinischen Vorrichtung besteht darin, dass bei der Komprimierung und vor allem bei der Expansion zumindest abschnittsweise eine Torsion der Gitterstruktur 10 erreicht wird. Da sich die in Längsrichtung benachbart bzw. gegenüberliegend angeordneten Verbindungsstellen 23, 24 bei der Zustandsänderung der Gitterstruktur 10, insbesondere beim Übergang vom expandierten Zustand zum komprimierten Zustand, zueinander versetzen bzw. unterschiedlich um die Längsachse der Gitterstruktur 10 rotieren, werden die nachfolgenden Umfangssegmente 20 insgesamt verdreht. Die Verdrehung bzw. Torsion erfolgt auch beim Übergang der Gitterstruktur 10 vom komprimierten in den expandierten Zustand, wobei die in Längsrichtung gegenüberliegend angeordneten Verbindungsstellen 23, 24 aus der versetzten Position gemäß Fig. 2 in die ausgerichtete Position gemäß Fig. 1 zurückgedreht werden. In beiden Fällen rotieren also einzelne Umfangssegmente 20 der Gitterstruktur 10 um die Längsachse der Gitterstruktur 10. Die Expansion der Gitterstruktur 10 erfolgt daher nicht durch eine geradlinig radiale Aufweitung, sondern im Wesentlichen schraubenlinienförmig. Dadurch wird erreicht, dass sich die Gitterstruktur 10 beim Aufweiten innerhalb eines Blutgefäßes 30 schraubenförmig in ein Blutgerinnsel 31 einschneidet, wie in Fig. 4 dargestellt. Die Stege 11, 12, 13, 14 bewegen sich nicht nur in radialer Richtung bezogen auf die Längsachse der Gitterstruktur 10 in das Blutgerinnsel 31 hinein, sondern weisen auch eine Umfangsrichtungskomponente auf, wobei durch die Umfangsbewegung der Stege 11, 12, 13, 14 im Blutgerinnsel 31 ein Hinterschnitt ausgebildet wird, der zu einer verbesserten Anhaftung des Blutgerinnsels 31 an der Gitterstruktur 10 führt. Die Gitterstruktur 10 wird in dem Blutgerinnsel 31 regelrecht verankert, so dass ein ungewolltes Ablösen des Blutgerinnsels 31 von der Gitterstruktur 10 vermieden wird.

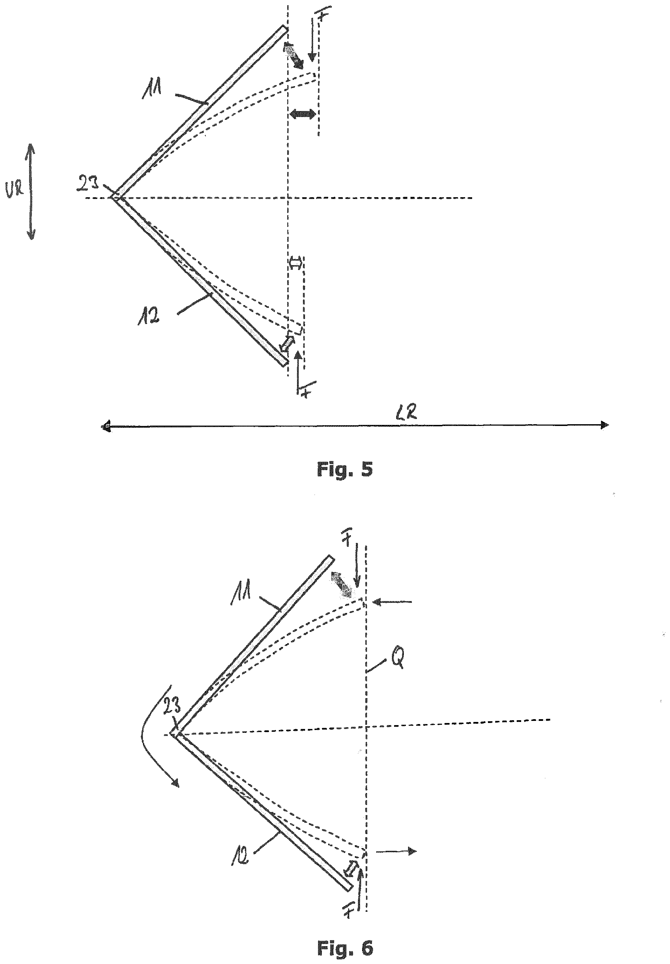

Zur Erläuterung der Funktionsweise der Gitterstruktur 10 bzw. allgemein der medizinischen Vorrichtung bzw. des Behandlungssystems, sind in den Fig. 5 und 6 jeweils zwei in Umfangsrichtung benachbart angeordnete und miteinander verbundene Stege 11, 12 gezeigt, wobei die Stege 11, 12 freigeschnitten sind, also für sich betrachtet werden. Zur weiteren Vereinfachung sind die Stege 11, 12 als Balken dargestellt. Die Flexibilität der Steg 11, 12 wird dadurch ermittelt, dass auf beide Stege 11, 12 dieselbe Kraft aufgebracht wird. Der Steg 11, 12, der sich stärker verformt als der andere Steg 12, 11, weist eine höhere Flexibilität im Sinne der Erfindung auf.

Die beiden Stege 11, 12 sind an einer Verbindungsstelle 23 miteinander verbunden. Bei der Balkendarstellung gemäß Fig. 5 und 6 weist der erste Steg 11 eine höhere Flexibilität als der zweite Steg 12 auf. Auf den ersten Steg 11 und den zweiten Steg 12 wird an den von der Verbindungsstelle 23 abgewandten Enden eine Kraft F in Umfangsrichtung UR der Gitterstruktur 10 aufgebracht. Dadurch werden die Stege 11, 12 ausgelenkt, wobei der Grad der Auslenkung unterschiedlich ist. Der erste Steg 11, der eine höhere Flexibilität aufweist, wird stärker ausgelenkt als der zweite Steg 12, der eine relativ niedrigere Flexibilität aufweist. Der ausgelenkte Zustand der Stege 11, 12 ist in Fig. 5 und 6 durch eine gestrichelte Kontur verdeutlicht. Die Auslenkung der Stege 11, 12 bewirkt eine Bewegung der axialen Enden der Stege 11, 12, die von der Verbindungsstelle 23 abgewandt sind, in Längsrichtung der Gitterstruktur 10. Da die Stege 11, 12 unterschiedliche Flexibilitäten aufweisen, wird das von der Verbindungsstelle 23 abgewandte axiale Ende des ersten Stegs 11, also das freie Ende des ersten Stegs 11, weiter in Längsrichtung LR der Gitterstruktur 10 verschoben als das freie Ende des zweiten Stegs 12. Dies ist in Fig. 5 durch waagrechte Doppelpfeile verdeutlicht. Diese Bewegung der Stege 11, 12 bzw. der freien Enden der Stege 11, 12 ist möglich, da bei der Prinzipdarstellung gemäß Fig. 5 die Lage der Verbindungsstelle 23 beibehalten wird. Überdies ist eine derartige Bewegung der Stege 11, 12 möglich, da die Annahme getroffen wird, dass die Stege 11, 12 freie Enden aufweisen. Dies ist bei der Gitterstruktur 10 der erfindungsgemäßen medizinischen Vorrichtung jedoch nicht der Fall.

Bei der Gitterstruktur 10 der erfindungsgemäßen medizinischen Vorrichtung bzw. des erfindungsgemäßen Behandlungssystems ist eine kreiszylinderförmige Geometrie der Gitterstruktur 10 vorausgesetzt. Die Umfangssegmente 20 bilden also geschlossene, im Wesentlichen kreiszylinderförmige Ringe. Mehrere Umfangssegmente 20 bilden die Gitterstruktur 10. Durch die kreiszylinderförmige Anordnung der Zellen 15 in den Umfangssegmenten 20 wird verhindert, dass sich die in Umfangsrichtung benachbart angeordneten Verbindungsstellen 21, 22 zueinander versetzen. Vielmehr besteht die geometrische Bedingung, dass die in Umfangsrichtung der Gitterstruktur benachbart angeordneten Verbindungsstellen eines Umfangssegments 20 in einer gemeinsamen Querschnittsebene Q angeordnet sind, die orthogonal auf der Längsachse der Gitterstruktur 10 steht. Tatsächlich erfolgt beim Übergang der Gitterstruktur 10 vom expandierten Zustand in den komprimierten Zustand eine Bewegung der in Längsrichtung benachbart angeordneten Verbindungsstellen 23, 24 entlang des Umfangs der Gitterstruktur 10. In Fig. 6 ist dieser Vorgang schematisch veranschaulicht, wobei die beiden freigeschnittenen Stege 11, 12 der Einfachheit halber als verformbare Balken dargestellt sind. Analog zu Fig. 5 ist der erste Steg 11 flexibler als der zweite Steg 12. Der verformte Zustand der Stege 11, 12 unter Einwirkung einer in Umfangsrichtung wirkenden Kraft F ist durch eine gestrichelte Kontur dargestellt. Daraus ist erkennbar, dass sich der erste Steg 11 beim Übergang vom expandierten Zustand zum komprimierten Zustand der Gitterstruktur 10 stärker verformt als der zweite Steg 12. Da die in der schematischen Darstellung gemäß Fig. 6 freien Enden der Stege 11, 12 aufgrund der geometrischen Bedingung auf einer gemeinsamen Querschnittsebene Q angeordnet sind, bewirkt die unterschiedliche Auslenkung bzw. Verformung der Stege 11, 12 eine Bewegung der Verbindungsstelle 23 auf dem Umfang der Gitterstruktur 10. Insbesondere rotiert die Verbindungsstelle 23 um einen Rotationsmittelpunkt, der innerhalb der Zelle 15 angeordnet ist. Da die Verbindungsstelle 23 gleichzeitig eine Verbindung zu einem weiteren Umfangssegment 20 der Gitterstruktur 10 bildet, wird das weitere Umfangssegment 20 insgesamt rotiert. Bezogen auf die gesamte Gitterstruktur 10 ergibt sich somit eine Torsion.

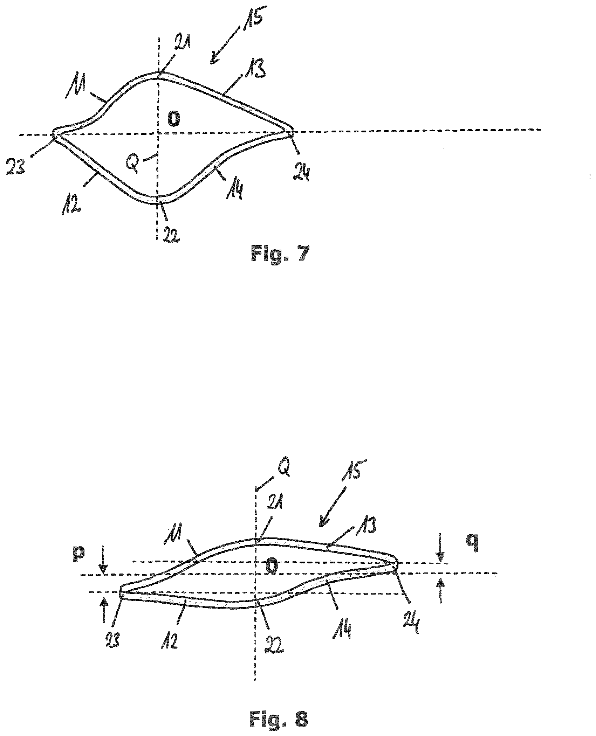

In den Fig. 7 und 8 ist nochmals verdeutlicht, wie sich der unterschiedliche Versatz der Verbindungsstellen 23, 24, die in Längsrichtung der Gitterstruktur 10 gegenüberliegend angeordnet sind, bei der Zustandsänderung der Gitterstruktur 10 einstellt. In Fig. 7 ist eine Zelle 15 der Gitterstruktur 10 im Ruhezustand dargestellt. Die Zelle 15 gemäß Fig. 7 entspricht im Wesentlichen der Zelle 15 gemäß Fig. 1. Insbesondere weist die Zelle 15 einen ersten Steg 11, einen zweiten Stege 12, einen dritten Steg 13 und einen vierten Steg 14 auf, wobei der erste und der vierte Steg 11, 14 eine höhere Flexibilität als der zweite und der dritte Steg 12, 13 aufweisen. Im Ruhezustand sind die Verbindungsstellen 21, 22, 23, 24 zwischen den einzelnen Stegen 11, 12, 13, 14 derart ausgerichtet, dass sie eine drachenviereckförmige Grundform der Zelle 15 aufspannen. Insbesondere sind die in Längsrichtung gegenüberliegend angeordneten Verbindungsstellen 23, 24 auf einer gemeinsamen Verbindungsgeraden angeordnet, die sich im Wesentlichen parallel zur Längsachse der Gitterstruktur 10 erstreckt und als Nullpunktgerade bezeichnet wird. Beim Übergang der Gitterstruktur vom expandierten Zustand in den komprimierten Zustand verändert sich die Position der in Längsrichtung gegenüberliegend angeordneten Verbindungsstellen 23, 24. Insbesondere versetzen sich die dritte Verbindungsstelle 23 und die vierte Verbindungsstelle 24 zueinander. Konkret werden die dritte Verbindungsstelle 23 und die vierte Verbindungsstelle 24 bezüglich der Nullpunktgeraden, die parallel zur Längsachse der Gitterstruktur 10 verläuft, ausgelenkt, wie in Fig. 8 dargestellt. Dabei weist die dritte Verbindungsstelle 23 einen höheren Auslenkgrad p als die vierte Verbindungsstelle 24 auf, die einen relativ kleineren Auslenkgrad q aufweist. Der Abstand der dritten Verbindungsstelle 23 von der Nullpunktsgeraden ist im komprimierten Zustand der Gitterstruktur 10 also größer als der Abstand der Verbindungsstelle 24 von der Nullpunktsgeraden. Die erste Verbindungsstelle 21 und die zweite Verbindungsstelle 22 sind in beiden Zuständen, also im expandierten Zustand gemäß Fig. 7 und im komprimierten Zustand gemäß Fig. 8, auf einer gemeinsamen Querschnittsebene Q angeordnet. Zwar kann sich die Querschnittsebene Q insgesamt in Längsrichtung der Gitterstruktur 10 bei der Zustandsänderung verschieben. Die einzelnen in Umfangsrichtung benachbart angeordneten Verbindungsstellen 21, 22 bleiben jedoch in jedem Zustand der Gitterstruktur 10 auf der gemeinsamen, ggf. verschobenen, Querschnittsebene Q.

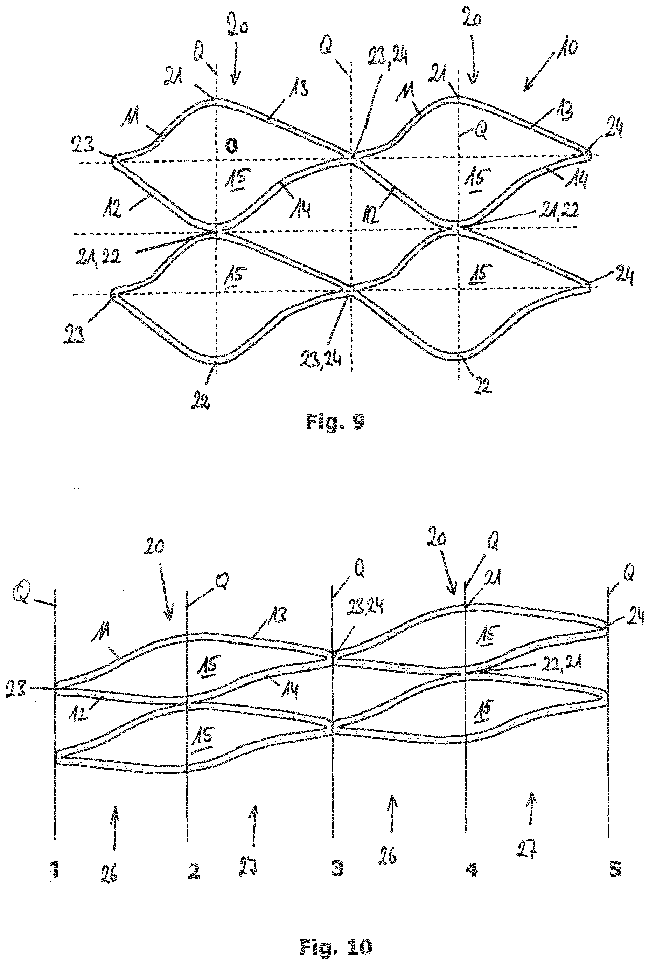

Fig. 9 zeigt einen Ausschnitt einer Gitterstruktur 10 mit insgesamt vier Zellen 15, wobei jeweils zwei Zellen 15 Teil eines Umfangssegments 20 sind. Innerhalb eines Umfangssegments 20 sind die Zellen 15 jeweils gleichartig ausgebildet. Bei dem Ausführungsbeispiel gemäß Fig. 9 sind die beiden dargestellten Umfangssegmente 20 gleichartig ausgebildet. Konkret sind die Zellen 15 gemäß Fig. 9 alle identisch. Die einzelnen Zellen 15 gemäß Fig. 9 entsprechen in ihrer Ausgestaltung der Zelle 15 gemäß Fig. 7. In Fig. 9 ist gut erkennbar, dass jede Verbindungsstelle 21, 22, 23, 24 jeweils vier Stege 11, 12, 13, 14 miteinander verbindet. Dabei kann jede Verbindungsstelle 21, 22, 23, 24 jeweils zwei Zellen 15 zugeordnet werden. Insbesondere bilden die in Umfangsrichtung gegenüberliegend angeordneten Verbindungsstellen 21, 22 jeweils eine erste Verbindungsstelle einer Zelle 15 und gleichzeitig eine zweite Verbindungsstelle 22 einer in Umfangsrichtung benachbarten Zelle 15. Die in Längsrichtung gegenüberliegend angeordneten Verbindungsstellen 23, 24 bilden jeweils eine dritte Verbindungsstelle 23 einer Zelle 15 und gleichzeitig eine vierte Verbindungsstelle 24 einer in Längsrichtung benachbart angeordneten Zelle 15. Durch gestrichelte, senkrechte Linien sind in Fig. 9 mehrere Querschnittsebenen Q eingezeichnet, die durch jeweils in Umfangsrichtung benachbart angeordnete Verbindungsstellen 21, 22, 23, 24 aufgespannt sind. Dabei wird ersichtlich, dass die dritten und vierten Verbindungsstellen 23, 24 nicht nur in Längsrichtung der Gitterstruktur 10 gegenüberliegend, sondern auch in Umfangsrichtung UR der Gitterstruktur 10 gegenüberliegend angeordnet sind. Beim Übergang der Gitterstruktur vom expandierten Zustand in den komprimierten Zustand und umgekehrt gilt also auch für die dritten und vierten Verbindungsstellen 23, 24, dass diese in jedem Zustand der Gitterstruktur 10 auf einer gemeinsamen Querschnittsebene Q angeordnet sind, wobei die Querschnittsebene Q die Längsachse der Gitterstruktur 10 unter einem rechten Winkel trifft. Die waagrechten gestrichelten Linien gemäß Fig. 9 verdeutlichen, dass die in Längsrichtung LR der Gitterstruktur 10 benachbart angeordneten Verbindungsstellen 21, 22, 23, 24 im Ruhezustand gemeinsam mit der Längsachse der Gitterstruktur 10 fluchten. Dabei wird deutlich, dass dies auch für die ersten und zweiten Verbindungsstellen 21, 22 gilt, die in einem Ruhezustand eine gemeinsame Nullpunktgerade umfassen können.

Fig. 10 zeigt den komprimierten Zustand des Gitterstrukturausschnitts gemäß Fig. 9. Die Querschnittsebenen, die die in Umfangsrichtung benachbart angeordneten Verbindungsstellen 21, 22, 23, 24 umfassen, sind in Fig. 10 als durchgezogene senkrechte Linien dargestellt. Es ist zu erkennen, dass die in Umfangsrichtung benachbart angeordneten Verbindungsstellen 21, 22, 23, 24 auch im komprimierten Zustand eine gemeinsame Querschnittsebene Q aufspannen. Ferner ist Fig. 10 zu entnehmen, dass die in Längsrichtung der Gitterstruktur 10 benachbart angeordneten Verbindungsstellen 21, 22, 23, 24 bezüglich einer Nullpunktgeraden versetzt sind. Insbesondere sind die beiden Umfangssegmente 20 zueinander versetzt bzw. in Umfangsrichtung UR der Gitterstruktur 10 verschoben. Aus Fig. 10 ist überdies leicht erkennbar, dass die Umfangssegmente 20 jeweils zwei Teilsegmente 26, 27 umfassen. Die Teilsegmente 26, 27 werden jeweils durch die Querschnittsebenen Q begrenzt.

Jedes Teilsegment 26, 27 weist mäanderförmig bzw. zickzackförmig angeordnete Stege 11, 12, 13, 14 auf. Jeder zweite Steg eines Teilsegments 26, 27 weist dabei eine höhere Flexibilität als der jeweils andere Steg auf. Beispielsweise weist das erste Teilsegment 26 eine Vielzahl erster Steg 11 und zweiter Stege 12 auf, wobei in Umfangsrichtung UR der Gitterstruktur 10 jeweils ein zweiter Steg 12 auf einem ersten Steg 11 folgt. Die Stege 11, 12, 13, 14 eines Teilsegments 26, 27 sind an Verbindungsstellen miteinander gekoppelt, so dass sich eine mäanderförmige Struktur ergibt. Bei dem ersten Teilsegment 26 ist vorgesehen, dass die ersten Stege 11 jeweils eine höhere Flexibilität als die zweiten Stege 12 aufweisen. Analoges gilt für das zweite Teilsegment 27, das eine Vielzahl dritter Stege 13 und vierter Stege 14 umfasst. Die dritten und vierten Stege 13, 14 sind an Verbindungsstellen 21, 22, 23, 24 miteinander gekoppelt derart, dass sich eine mäanderförmige Struktur ergibt. Jeder zweite Steg 13, 14 weist eine höhere Flexibilität als der jeweils andere Steg auf. Insbesondere ist bei dem zweiten Teilsegment 27 vorgesehen, dass die vierten Stege 14 jeweils eine höhere Flexibilität als die dritten Stege 13 aufweisen.

In Längsrichtung der Gitterstruktur 10 wechseln sich jeweils erste und zweite Teilsegmente 26, 27 ab. Bei dem Ausführungsbeispiel gemäß Fig. 10 ist überdies vorgesehen, dass die zweiten Teilsegmente 27 eine größere Breite als die ersten Teilsegmente 26 aufweisen. Mit anderen Worten weisen die zweiten Teilsegmente 27 Stege 13, 14 auf, die länger sind als die Stege 11, 12 des ersten Teilsegments 26.

Durch die unterschiedlichen Flexibilitäten der einzelnen Stege 11, 12, 13, 14 wird bewirkt, dass sich die ersten und zweiten Teilsegmente 26, 27 im Wesentlichen gegeneinander verdrehen bzw. gegeneinander in Umfangsrichtung der Gitterstruktur 10 verschieben. Über die gesamte Gitterstruktur 10 hinweg ergibt sich somit die erfindungsgemäße Torsion.

Für alle Zustände der Gitterstruktur 10, also für den expandierten Zustand, den komprimierten Zustand und alle möglichen Zwischenzustände, gilt nicht nur, dass die in Umfangsrichtung UR benachbart angeordneten Verbindungsstellen eine gemeinsame Querschnittsebene Q aufspannen, die zur Längsachse der Gitterstruktur 10 orthogonal ausgerichtet ist, sondern auch, dass der Abstand der in Umfangsrichtung UR benachbart angeordneten Verbindungsstellen innerhalb derselben Querschnittsebene Q gleich ist. Insbesondere entspricht der Abstand zwischen zwei Verbindungsstellen 21, 22, 23, 24, die in Umfangsrichtung UR benachbart angeordnet sind, einem mathematischen Bruchteil des Umfangs der Gitterstruktur 10. Der Abstand zwischen den Verbindungsstellen 21, 22, 23, 24 einer gemeinsamen Querschnittsebene Q ist also einheitlich.

Bei den zuvor beschriebenen Ausführungsbeispielen ist vorgesehen, dass in einem expandierten Zustand der Gitterstruktur 10 die in Längsrichtung LR der Gitterstruktur 10 benachbart angeordneten Verbindungsstellen 23, 24 auf einer gemeinsamen Linien angeordnet sind, die sich parallel zur Längsachse der Gitterstruktur 10 erstreckt. Alternativ ist es möglich, dass die in Längsrichtung benachbart angeordneten Verbindungsstellen 23, 24 in einem anderen Zustand der Gitterstruktur auf dieser Nullpunktsgeraden angeordnet sind. Die dritte und vierte Verbindungsstelle 23, 24 können also im expandierten Zustand der Gitterstruktur 10 zueinander versetzt angeordnet bzw. aus der Nullpunktsgeraden ausgelenkt sein. Es ist auch möglich, dass die dritte und vierte Verbindungsstelle 23, 24 sowohl im expandierten Zustand, als auch im komprimierten Zustand aus der Nullpunktsgeraden ausgelenkt sind. Die Anordnung auf der Nullpunktsgeraden kann sich in einem Zwischenzustand der Gitterstruktur 10 einstellen. Ferner kann vorgesehen sein, dass die Gitterstruktur 10 derart geformte Zellen 15 aufweist, dass die in Längsrichtung benachbart angeordneten Verbindungsstellen 23, 24 in keinem Zustand der Gitterstruktur 10 auf einer Nullpunktsgeraden angeordnet sind.

Als Nullpunktsgerade wird im Rahmen der Anmeldung die Gerade bezeichnet, die einerseits die in Längsrichtung benachbart angeordneten Verbindungsstellen 23, 24 einer Zelle 15 verbindet und andererseits parallel zur Längsachse der Gitterstruktur 10 bzw. senkrecht zur Querschnittsebene Q ausgerichtet ist. Wenn die in Längsrichtung benachbart angeordneten Verbindungsstellen 23, 24 auf der Nullpunktsgeraden positioniert sind, deckt sich eine Verbindungslinie zwischen den in Längsrichtung benachbart angeordneten Verbindungsstellen 23, 24 mit der Nullpunktsgeraden. Es ist möglich, dass sich die Verbindungslinie zwischen den in Längsrichtung benachbart angeordneten Verbindungsstellen 23, 24 einer Zelle in keinem Zustand der Gitterstruktur 10 mit der Nullpunktsgeraden deckt. Vielmehr kann die Verbindungslinie der in Längsrichtung benachbart angeordneten Verbindungsstellen 23, 24 in jedem Zustand der Gitterstruktur 10 die Nullpunktsgerade unter einem Winkel schneiden.

Die medizinische Vorrichtung nach einem der zuvor genannten Ausführungsbeispiele ist vorzugsweise Teil eines Behandlungssystems, das ferner einen Führungsdraht umfasst, der mit einem proximalen Ende der Vorrichtung, insbesondere der Gitterstruktur 10, verbunden ist. Der Führungsdraht ist längsverschieblich in einem Katheter angeordnet. Mit Hilfe des Führungsdrahts kann die Gitterstruktur 10 bzw. allgemein die medizinische Vorrichtung, im komprimierten Zustand durch den Katheter an einen Behandlungsort geführt werden. Die Bewegung der Gitterstruktur 10 durch den Katheter erfolgt dabei durch Schieben des proximalen Endes des Führungsdrahts in distale Richtung durch den Katheter. Am Behandlungsort wird die Gitterstruktur 10 bzw. allgemein die medizinische Vorrichtung freigesetzt. Das Freisetzen der Gitterstruktur 10 kann auf unterschiedliche Weise erfolgen. Einerseits kann der Führungsdraht weiter in distale Richtung geschoben werden, wobei der Katheter ortsfest gehalten wird. Alternativ kann der Führungsdraht ortsfest gehalten werden und der Katheter in proximale Richtung zurückgezogen werden, um die Gitterstruktur 10 aus dem Katheter zu entlassen. In beiden Fällen erfolgt die Entlassung der Gitterstruktur 10 durch eine translatorische Relativbewegung zwischen dem Führungsdraht und dem Katheter.

Durch Wegfall des äußeren Zwanges, den der Katheter auf die Gitterstruktur ausübt und der diese im komprimierten Zustand hält, expandiert die Gitterstruktur 10. Dazu weist die Gitterstruktur 10 vorzugsweise ein selbstexpandierendes Material auf. Derartige Materialien sind beispielsweise Formgedächtnislegierungen, insbesondere Nickel-Titan-Legierungen.

Aufgrund der zuvor beschriebenen Konstruktion und Eigenschaften der Gitterstruktur 10 erfolgt allein durch die radiale Expansion der Gitterstruktur 10 eine Torsion. Die Drehung der Gitterstruktur 10 wird also durch die translatorische Relativbewegung zwischen der Gitterstruktur 10 bzw. den damit verbundenen Führungsdraht und dem Katheter bewirkt. Die translatorische Relativbewegung zwischen Führungsdraht und Katheter ist vergleichsweise einfach steuerbar im Gegensatz zu einer manuellen Drehung, die am proximalen Ende des Führungsdrahts durchgeführt wird. Bei der manuellen Drehung des Führungsdrahts erfolgt zunächst eine Torsion des Führungsdrahts, so dass die eigentliche Verdrehung der distal angeordneten medizinischen Vorrichtung bzw. Gitterstruktur 10 verzögert erfolgt. Dies ist bei einer translatorischen Bewegung nicht gegeben, so dass die Handhabbarkeit des erfindungsgemäßen Behandlungssystems verbessert ist.

### Bezugszeichenliste

- 10: Gitterstruktur
- 11: erster Steg
- 12: zweiter Steg
- 13: dritter Steg
- 14: vierter Steg
- 15: Zelle
- 20: Umfangssegment
- 21: erste Verbindungsstelle
- 22: zweite Verbindungsstelle
- 23: dritte Verbindungsstelle
- 24: vierte Verbindungsstelle
- 26: erstes Teilsegment
- 27: zweites Teilsegment
- 30: Blutgefäß
- 31: Blutgerinnsel

## Patentansprüche

1. Medizinische Vorrichtung mit einer komprimierbaren und expandierbaren, kreiszylinderförmigen Gitterstruktur (10), die Umfangssegmente (20) aus geschlossenen Zellen (15) umfasst, wobei die Zellen (15) durch jeweils vier Stege (11, 12, 13, 14) begrenzt sind, die an Verbindungsstellen (21, 22, 23, 24) miteinander gekoppelt sind und von denen
- jeweils zwei in Umfangsrichtung UR der Gitterstruktur (10) benachbarte und an einer Verbindungsstelle (21, 22) miteinander gekoppelte Stege (11, 12, 13, 14) unterschiedlich flexibel sind derart, dass der Steg (11, 13) mit höherer Flexibilität beim Übergang der Gitterstruktur (10) vom expandierten Zustand in den komprimierten Zustand stärker verformbar als der Steg (12, 13) mit niedrigerer Flexibilität ist, und von denen
- die Stege (11, 13) mit höherer Flexibilität und die Stege (12, 14) mit niedrigerer Flexibilität diagonal jeweils gegenüberliegend angeordnet sind derart, dass zwei in Längsrichtung LR der Gitterstruktur (10) gegenüberliegend angeordnete Verbindungsstellen (23, 24) der Zelle (15) beim Übergang der Gitterstruktur (10) vom expandierten Zustand in den komprimierten Zustand in Umfangsrichtung UR gegenläufig zueinander versetzt werden,
wobei alle Zellen (15) eines Umfangssegments (20) gleichartig ausgebildet sind derart, dass die gesamte Gitterstruktur (10) beim Übergang vom expandierten Zustand in den komprimierten Zustand zumindest abschnittsweise tordiert, und wenigstens zwei benachbarte Umfangssegmente (20) gleichartig ausgebildet sind,
**dadurch gekennzeichnet, dass**
in Umfangsrichtung UR benachbart angeordnete Verbindungsstellen (23, 24) eines Umfangssegments (20) eine gemeinsame Querschnittsebene Q der Gitterstruktur (10) aufspannen, die orthogonal zur Längsachse der Gitterstruktur (10) angeordnet ist, wobei sich die in Umfangsrichtung UR benachbart angeordneten Verbindungsstellen (23, 24) beim Übergang der Gitterstruktur (10) vom expandierten Zustand in den komprimierten Zustand in der Querschnittsebene Q bewegen.

2. Vorrichtung nach Anspruch 1,
**dadurch gekennzeichnet, dass**
jede Zelle (15) vier Verbindungsstellen (21, 22, 23, 24) umfasst, die im expandierten Zustand der Gitterstruktur (10) eine drachenviereckförmige Grundform der Zelle (15) aufspannen.

3. Vorrichtung nach einem der Ansprüche 1-2,
**dadurch gekennzeichnet, dass**
in Längsrichtung LR benachbart angeordnete Stege (11, 12, 13, 14) jeweils unterschiedliche Steglängen aufweisen.

4. Vorrichtung nach einem der Ansprüche 1-3,
**dadurch gekennzeichnet, dass**
die Stege (11, 13) mit höherer Flexibilität im Wesentlichen S-förmig und die Stege (12, 14) mit niedrigerer Flexibilität geradlinig ausgebildet sind.

5. Vorrichtung nach einem der Ansprüche 1-4,
**dadurch gekennzeichnet, dass**
die Stege (11, 13) mit höherer Flexibilität eine Stegbreite aufweisen, die kleiner als die Stegbreite der Stege (12, 14) mit niedrigerer Flexibilität ist.

6. Vorrichtung nach einem der Ansprüche 1-5,
**dadurch gekennzeichnet, dass**
die Stege (11, 13) mit höherer Flexibilität Biegestellen, insbesondere Verjüngungen oder Verbreiterungen oder Durchbrüche, aufweisen.

7. Vorrichtung nach einem der Ansprüche 1-6,
**dadurch gekennzeichnet, dass**
die Stege (11, 12, 13, 14) an den Verbindungsstellen (21, 22, 23, 24) einstückig verbunden sind.

8. Vorrichtung nach einem der Ansprüche 1-7,
**dadurch gekennzeichnet, dass**
an den Verbindungsstellen (21, 22, 23, 24) jeweils vier Stege (11, 12, 13, 14) miteinander verbunden sind.

9. Vorrichtung nach einem der Ansprüche 1-8,
**dadurch gekennzeichnet, dass**
die in Längsrichtung LR der Gitterstruktur (10) gegenüberliegend angeordneten Verbindungsstellen (23, 24) einen unterschiedlichen Versetzungsgrad beim Übergang der Gitterstruktur (10) vom expandierten in den komprimierten Zustand aufweisen.

10. Vorrichtung nach einem der Ansprüche 1-9,
**dadurch gekennzeichnet, dass**
die Umfangssegmente (20) jeweils zwei Teilsegmente (26, 27) umfassen, die jeweils mäanderförmig angeordnete Stege (11, 12, 13, 14) aufweisen, wobei in Längsrichtung der Gitterstruktur (10) unmittelbar benachbarte Teilsegmente (26, 27) zumindest im komprimierten Zustand der Gitterstruktur (10) unterschiedliche Teilsegmentbreiten aufweisen.

11. Behandlungssystem mit einer medizinischen Vorrichtung gemäß einem der Ansprüche 1 bis 10 und mit einem Katheter, in dem ein Führungselement längsverschieblich angeordnet ist, wobei das Führungselement fest, insbesondere drehfest, mit einem axialen Ende der Gitterstruktur (10) der medizinischen Vorrichtung verbunden ist.

## Claims

1. A medical device having a compressible and expandable circular cylindrical mesh structure (10), comprising circumferential segments (20) made of closed cells (15), wherein the cells (15) are each bordered by four webs (11, 12, 13, 14), which are coupled to one another at the connecting points (21, 22, 23, 24), and of which
- two webs (11, 12, 13, 14) that are neighboring one another in the circumferential direction UR of the mesh structure (10) and are coupled to one another at a connecting point (21, 22) are flexible to different extents, such that the web (11, 13) having the greater flexibility can be deformed in the transition of the mesh structure (10) from the expanded state to the compressed state to a greater extent than the web (12, 13) having a lower flexibility, and of which
- the webs (11, 13) having the greater flexibility and the webs (12, 14) having the lower flexibility are each arranged diagonally opposite one another, in such a way that two connecting points (23, 24) of the cell (15) arranged opposite one another in the longitudinal direction LR of the mesh structure (10) are dislocated in opposed directions from one another in the circumferential direction UR in the transition of the mesh structure (10) from the expanded state to the compressed state,
wherein all the cells (15) of a circumferential segment (20) are designed similarly, such that the entire mesh structure (10) is twisted in at least some sections in the transition from the expanded state to the compressed state, and at least two neighboring circumferential segments (20) are designed similarly,
**characterized in that**
connecting points (23, 24) of a circumferential segment(20) arranged next to one another in the circumferential direction UR span a common cross-sectional plane Q of the mesh structure (10) arranged orthogonally to the longitudinal axis of the mesh structure (10), wherein the connecting points (23, 24) arranged next to one another in the circumferential direction UR move in the cross-sectional plane Q in the transition of the mesh structure (10) from the expanded state to the compressed state.

2. The device according to claim 1,
**characterized in that**
each cell (15) compromises four connecting points (21, 22, 23, 24), which in the expanded state of the mesh structure (10) span a kite-shaped basic shape of the cell (15).

3. The device according to any one of claims 1-2,
**characterized in that**
webs (11, 12, 13, 14) arranged next to one another in the longitudinal direction LR each have different web lengths.

4. The device according to any one of claims 1-3,
**characterized in that**
the webs (11, 13) having a greater flexibility are are designed to be essentially S-shaped, and the webs (12, 14) having a lower flexibility are designed to be essentially rectilinear.

5. The device according to any one of claims 1-4,
**characterized in that**
the webs (11, 13) having the greater flexibility have a web width less than the web width of the webs (12, 14) having the lower flexibility.

6. The device according to any one of claims 1-5,
**characterized in that**
the webs (11, 13) having the greater flexibility have bending points, in particular tapered or widened areas or breakthroughs.

7. The device according to any one of claims 1-6,
**characterized in that**
the webs (11, 12, 13, 14) are connected to the connecting points (21, 22, 23, 24) in one piece.

8. The device according to any one of claims 1-7,
**characterized in that**
four webs (11, 12, 13, 14) are each joined to one another at the connecting points (21, 22, 23, 24).

9. The device according to any one of claims 1-8,
**characterized in that**
the connecting points (23, 24) arranged opposite one another in the longitudinal direction LR of the mesh structure (10) have different degrees of dislocation in the transition of the mesh structure (10) from the expanded state to the compressed state.

10. The device according to any one of claims 1-9,
**characterized in that**
the circumferential segments (20) each comprise two partial segments (26, 27), each of which has webs (11, 12, 13, 14) arranged in a meandering pattern, wherein directly adjacent partial segments (26, 27) in the longitudinal direction of the mesh structure (10) have different partial segment widths, at least in the compressed state of the mesh structure (10).

11. A treatment system comprising a medical device according to any one of claims 1 to 10 and comprising a catheter, in which a guide element is arranged, so that it is longitudinally displaceable, wherein the guide element is connected fixedly, in particular in a rotationally fixed manner, to an axial end of the mesh structure (10) of the medical device.

## Revendications

1. Dispositif médical comprenant une structure en grille (10) cylindrique comprimable et extensible, qui comprend des segments périphériques (20) de cellules fermées (15), dans lequel les cellules (15) sont délimitées par respectivement quatre branches (11, 12, 13, 14) qui sont couplées entre elles en des points de liaison (21, 22, 23, 24) et desquels :
- respectivement deux branches (11, 12, 13, 14) adjacentes dans le sens périphérique UR de la structure en grille (10) et couplées entre elles en un point de liaison (21, 22) sont de flexibilité différente, de sorte que la branche (11, 13) avec une plus grande flexibilité, peut être plus déformée que la branche (12, 13) avec une moindre flexibilité lorsque la structure en grille (10) passe de l'état expansé à l'état comprimé, et desquels
- les branches (11, 13) d'une plus grande flexibilité et les branches (12, 14) d'une moindre flexibilité sont disposées de façon diagonale en étant respectivement opposées, que deux points de liaison (23, 24) des cellules (15) disposés opposés dans le sens longitudinal LR de la structure en grille (10) sont décalés l'un de l'autre dans le sens inverse dans le sens périphérique UR lorsque la structure en grille (10) passe de l'état expansé à l'état comprimé,
dans lequel toutes les cellules (15) d'un segment périphérique (20) sont formées de la même manière de sorte que la totalité de la structure en grille (10) se tord au moins par tronçons lors du passage de l'état expansé à l'état comprimé, et au moins deux segments périphériques voisins (20) sont formés de la même manière,
**caractérisé en ce que**
des points de liaison (23, 24) d'un segment périphérique (20) disposés adjacents dans le sens périphérique UR définissent un plan transversal Q commun de la structure en grille (10) qui est disposé de façon orthogonale à l'axe longitudinal de la structure en grille (10), dans lequel les points de liaison (23, 24) disposés adjacents dans le sens périphérique UR se déplacent dans le plan transversal Q lorsque la structure en grille (10) passe de l'état expansé à l'état comprimé.

2. Dispositif selon la revendication 1, **caractérisé en ce que** chaque cellule (15) comprend quatre points de liaison (21, 22, 23, 24) qui définissent une forme de base en cerf-volant des cellules (15) lorsque la structure en grille (10) est dans l'état expansé.

3. Dispositif selon l'une des revendications 1 - 2, **caractérisé en ce que** des branches (11, 12, 13, 14) disposées adjacentes dans le sens longitudinal LR présentent des longueurs de branches différentes.

4. Dispositif selon l'une des revendications 1 - 3, **caractérisé en ce que** les branches (11, 13) avec une plus grande flexibilité sont essentiellement en S et les branches (12, 14) avec une moindre flexibilité sont droites.

5. Dispositif selon l'une des revendications 1 - 4, **caractérisé en ce que** les branches (11, 13) avec une plus grande flexibilité présentent une largeur de branche qui est inférieure à la largeur des branches (12, 14) avec une moindre flexibilité.

6. Dispositif selon l'une des revendications 1 - 5, **caractérisé en ce que** les branches (11, 13) avec une plus grande flexibilité présentent des points de flexion, en particulier des rétrécissements ou des élargissements ou des interruptions.

7. Dispositif selon l'une des revendications 1 - 6, **caractérisé en ce que** les branches (11, 12, 13, 14) sur les points de liaison (21, 22, 23, 24) sont fabriquées d'une seule pièce.

8. Dispositif selon l'une des revendications 1 - 7, **caractérisé en ce que** respectivement quatre branches (11, 12, 13, 14) sont reliées entre elles sur les points de liaison (21, 22, 23, 24).

9. Dispositif selon l'une des revendications 1 - 8, **caractérisé en ce que** les points de liaison (23, 24) disposés opposés dans le sens longitudinal LR de la structure en grille (10) présentent un degré de déplacement différent lorsque la structure en grille (10) passe de l'état expansé à l'état comprimé.

10. Dispositif selon l'une des revendications 1 - 9, **caractérisé en ce que** les segments périphériques (20) comprennent respectivement deux segments partiels (26, 27) qui présentent respectivement des branches (11, 12, 13, 14) disposées sinueuses, dans lequel, dans le sens longitudinal de la structure en grille (10), des segments partiels (26, 27) directement voisins présentent différentes largeurs de segment partiel au moins lorsque la structure en grille (10) est dans l'état comprimé.

11. Système de traitement comprenant un dispositif médical selon l'une des revendications 1 à 10 et comprenant un cathéter dans lequel un élément de guidage est disposé en étant mobile longitudinalement, dans lequel l'élément de guidage est relié fixement, en particulier fixe en rotation, avec une extrémité axiale de la structure en grille (10) du dispositif médical.
